# EUROPÄISCHE PATENTSCHRIFT

(11) **EP 1 618 915 B1**
(45) Veröffentlichungstag und Bekanntmachung des Hinweises auf die Patenterteilung: **31.01.2007**
(21) Anmeldenummer: 04017195.1
(22) Anmeldetag: 21.07.2004
(51) Int. Cl.: A61M 37/00

(54) **Vorrichtung zum lokalen Aufstechen einer Haut**
Device to puncture the skin
Dispositif pour la ponction de la peau

(43) Veröffentlichungstag der Anmeldung: 25.01.2006
(73) Patentinhaber: MediUm-TECH Medizingeräte GmbH, 14167 Berlin (DE)
(72) Erfinder: Lisec, Walter, 13053 Berlin (DE)
(74) Vertreter: Bittner, Thomas L.

(56) Entgegenhaltungen:
- DE-A- 10 343 590
- DE-U- 29 919 199
- US-A- 5 472 449
- US-B1- 6 588 301

## Beschreibung

Die Erfindung bezieht sich auf eine Vorrichtung zum lokalen Aufstechen einer Haut, insbesondere zum Aufbringen von permanentem Make-up oder eines Tattoos.

Eine derartige Vorrichtung ist beispielsweise aus dem Dokument US 6,505,530 B2 bekannt. Bei der bekannten Vorrichtung wird in einem Gehäuse eine Nadel geführt, so daß die Nadel zwischen einer ausgefahrenen Stellung, in welcher eine Nadelspitze außerhalb einer Öffnung an dem Gehäuse angeordnet ist, und einer eingefahrenen Stellung verlagerbar ist, in welcher die Nadelspitze in dem Gehäuse angeordnet ist. Mittels repetierender Vorwärts- und Rückwärtsbewegungen, wodurch sich die Nadel zwischen der ausgefahrenen und der eingefahrenen Stellung bewegt, wird die Nadel wiederholt in die Haut gestochen und wieder herausgezogen, so daß ein aufzubringendes Medium in flüssiger Form in die Haut eindringen kann. Bei Verwendung der bekannten Vorrichtung zum Aufbringen von permanentem Make-up oder eines Tattoos wird ein Farbstoff in die Haut eingebracht. Der Farbstoff kann hierbei aufgebracht werden, indem eine Gehäusespitze in ein Farbstoffreservoir getaucht wird, so daß Farbstoff sich entlang der Nadel verteilt, gegebenenfalls bis in das Gehäuse hinein, und beim Betreiben der Vorrichtung dann in den aufgestochenen Hautbereich gelangt. Alternativ oder ergänzend kann vorgesehen sein, daß am Gehäuse der Vorrichtung ein Farbstofftank angebracht ist, welcher den Nachschub von Farbstoff in das Innere des Gehäuses in Kontakt mit der vor- und zurückbewegten Nadel gewährleistet.

Der Antrieb der Nadel beim Vorwärtsbewegen wird bei der bekannten Vorrichtung mit Hilfe einer Antriebseinrichtung erreicht, die einen Motor umfaßt. Das Gehäuse mit der geführten Nadel kann als separates Modul ausgeführt sein, welches lösbar an die Antriebseinrichtung koppelbar ist. Es kann aber auch vorgesehen sein, daß das Gehäuse mit der geführten Nadel und die Antriebseinrichtungen in eine Vorrichtung integriert sind. Es sind andere Vorrichtungen bekannt, bei denen die Nadel vom Gehäuse abgenommen werden kann.

Um das Vordringen von Flüssigkeit, sowohl Farbstoff als auch Körperflüssigkeit, die beim Aufstechen der Haut freigesetzt wird, in den Bereich, in welchem die Antriebseinrichtung angekoppelt ist, oder in die Antriebseinrichtung selbst zu verhindern, ist bei der bekannten Vorrichtung in dem Gehäuse eine Membran aus einem elastisch dehnbaren Material angeordnet. Mit Hilfe der Membran werden in dem Gehäuse ein vorderseitiger Raum auf einer Vorderseite der Membran und ein rückseitiger Raum auf einer Rückseite der Membran getrennt. Die Membran dient zum dichten Trennen der beiden Räume des Gehäuses. Im Dokument US 6,505,530 B2 sind Ausführungen beschrieben, bei denen die Nadel durch einen Durchbruch in der Membran hindurch gebildet ist, wobei die Nadel im Bereich des Durchbruchs von einer Dichtlippe umgriffen wird, so daß die Nadel beim Verlagern zwischen der ausgefahrenen und der eingefahrenen Stellung in dem Durchbruch rutscht und die Dichtlippe der Membran hierbei auf der Nadel gleitet. Beim Betrieb der bekannten Vorrichtung zum lokalen Aufstechen der Haut wird die Nadel mit Hilfe einer von der Antriebseinrichtung erzeugten Antriebskraft aus der eingefahrenen in die ausgefahrene Stellung gebracht. Das Zurückholen der Nadel aus der ausgefahrenen Stellung in die eingefahrene Stellung wird mit Hilfe einer Federkraft erreicht, die von einer in dem Gehäuse angeordneten Spiralfeder erzeugt und über den Nadelschaft auf die Nadel eingeleitet wird.

Es sind auch Vorrichtungen zum lokalen Aufstechen einer Haut, insbesondere zum Aufbringen von permanentem Make-up oder eines Tattoos, bekannt, bei denen die Nadelspitze in der eingefahrenen Stellung nicht in dem umgebenden Gehäuse aufgenommen ist, sondern noch übersteht.

Der Anmelder hat sich unter Bezugnahme auf DE 103 43 590 A1 freiwillig eingeschränkt und gesonderte Patentansprüche für Deutschland vorgelegt.

Aufgabe der Erfindung ist es, eine verbesserte Vorrichtung zum lokalen Aufstechen einer Haut, insbesondere zum Aufbringen von permanentem Make-up oder eines Tattoos, anzugeben, die über einen vereinfachten mechanischen Aufbau verfügt.

Diese Aufgabe wird bei einer Vorrichtung nach dem Oberbegriff des Anspruchs 1 erfindungsgemäß dadurch gelöst, daß mit Hilfe eines Kopplungsmechanismus die Nadel und die Membran für eine Rückbewegung der Nadel in Richtung der eingefahrenen Stellung fest miteinander gekoppelt sind, wobei über den Kopplungsmechanismus eine von der Membran erzeugte Rückholkraft zum Zurückbewegen der Nadel in Richtung der eingefahrenen Stellung auf die Nadel eingeleitet wird.

Ein wesentlicher Vorteil, welcher mit der Erfindung gegenüber dem Stand der Technik erreicht wird, besteht darin, daß die bei bekannten Vorrichtungen vorgesehene Feder zum Ausbilden der Rückholkraft zum Zurückbewegen der Nadel in Richtung der eingefahrenen Stellung eingespart wird. Die Rückholkraft wird mit Hilfe der Membran zur Verfügung gestellt, wenn diese gedehnt ist, so daß die Membran neben der Trennfunktion in dem Gehäuse eine weitere Funktion erfüllt, nämlich das Zurückholen der Nadel aus der ausgefahrenen Stellung. Zu diesem Zweck sind die Nadel und die Membran über den Kopplungsmechanismus fest miteinander verbunden, was bedeutet, daß Nadel und ein Kopplungsabschnitt der Membran, an welchen die Nadel gekoppelt ist, beim Zurückziehen der Membran relativ zueinander in ihrer Position fixiert sind, so daß die Nadel insbesondere rutschfest an der Membran befestigt ist, im Unterschied zu der bekannten Vorrichtung, bei der die Nadel oder der Nadelschaft gleitend in einem Durchbruch der Membran gelagert ist. Auf diese Weise wird nicht nur die Anzahl der bei der Herstellung der Vorrichtung zusammen zu fügenden Bauteile vermindert, sondern auch die Konstruktion vereinfacht.

Im Vergleich zu der bekannten Vorrichtung, bei der die Nadel mit Hilfe einer Feder zurück bewegt wird, besteht weiterhin der Vorteil, daß Geräusche vermindert sind, die beim Betrieb der Vorrichtung entstehen.

Eine mechanisch einfache Möglichkeit zum Koppeln der Nadel an die Membran für die Einleitung der Rückholkraft auf die Nadel ist bei einer bevorzugten Ausführungsform der Erfindung dadurch gebildet, daß an der Nadel ein Nadelschaft gebildet ist und die von der Membran erzeugte Rückholkraft über den Nadelschaft auf die Nadel eingeleitet wird. Der Nadelschaft bildet ein hinsichtlich seines konstruktiven Aufbaus selbständig optimierbares Teil und ist mit der Nadel starr verbunden.

Bei einer Ausgestaltung der Erfindung ist vorgesehen, daß der Kopplungsmechanismus an der Membran gebildete Montagemittel zum Halten der Nadel auf der Vorderseite der Membran umfaßt, in welchen ein Ende der Nadel montiert ist.

Die feste Kopplung zwischen Membran und Nadel kann alternativ auch über ein Antriebsbauteil erfolgen, bei dem es sich beispielsweise um eine Verlängerung handelt und welches Teil des Antriebsmechanismus ist, mit dem die Antriebskraft für den Nadelvortrieb auf die Nadel übertragen wird. Hierbei koppeln Antriebsbauteil und Membran, wobei die Nadel für die Rückbewegung starr mit dem Antriebsbauteil verbunden ist, wahlweise unter Zwischenschaltung des Nadelschafts. Das Antriebsbauteil wird aufgrund der von der Membran erzeugten und mittels Kopplung in das Antriebsbauteil eingeleiteten Rückholkraft zurück bewegt und hierdurch die Nadel.

Eine zweckmäßige Ausgestaltung der Erfindung sieht vor, daß der vorderseitige Raum auf der Vorderseite der Membran und der rückseitige Raum auf der Rückseite der Membran mit Hilfe der Membran fluiddicht getrennt sind. Auf diese Weise wird gewährleistet, daß weder das Medium, welches mittels des lokalen Aufstechens der Haut eingebracht wird, noch Körperflüssigkeit aus dem Bereich, in welchem die Nadel in dem Gehäuse geführt wird, auf die Rückseite der Membran gelangen können, wo die Ankopplung zur Antriebseinrichtung erfolgt. Der rückseitige Raum kann hierbei auch in der Antriebseinrichtung gebildet sein, wenn die Membran als rückseitiger Abschluß des Gehäuses gebildet ist.

Die Membran weist dehnbare Abschnitte auf, die in einer nicht gedehnten Grundform der Membran im wesentlichen in Längsrichtung der Nadel gebildet sind. Bei dieser Ausführungsform wird die Rückholkraft zum Zurückbewegen der Nadel in Richtung der eingefahrenen Stellung wesentlich aufgrund der dehnbaren Abschnitte in Längsrichtung der Nadel erzeugt, so daß die Rückholkräfte im wesentlichen in Längsrichtung der Nadel wirken, was die Effizienz ihrer Wirkung verbessert.

In Längsrichtung der Nadel gebildete dehnbare Abschnitte der Membran werden bei einer bevorzugten Ausführungsform der Erfindung dadurch gebildet, daß ein Querschnitt der Membran in Längsrichtung U-förmig oder V-förmig ist.

Bevorzugt ist die Membran lösbar an dem Gehäuse montiert, wodurch die Nutzung unterschiedlicher Membrane und/oder deren Verschleiß bedingter Austausch erleichtert wird.

Das Gehäuse kann als ein Modul ausgeführt sein, welches lösbar an ein Antriebsmodul koppelbar ist, wodurch Vorrichtungen mit unterschiedlichen Nadeln mit einem Antriebsmodul verwendet werden können. Des weiteren ist hierdurch die Möglichkeit geschaffen, verbrauchte Vorrichtungen mit der im Gehäuse geführten Nadel auszutauschen, wenn diese für eine weitere Nutzung unbrauchbar ist. Besonders bevorzugt wird in diesem Zusammenhang, daß das als Modul ausgeführte Gehäuse ein Einwegmodul ist.

Die Vorrichtung zum lokalen Aufstechen der Haut entfaltet die beschriebenen Vorteile bei der Verwendung zum Aufbringen von permanentem Make-up oder eines Tattoos auf die Haut.

Die Erfindung wird im folgenden in Form von Ausführungsbeispielen unter Bezugnahme auf eine Zeichnung näher erläutert. Hierbei zeigen:
- Fig. 1: eine Vorrichtung zum lokalen Aufstechen einer Haut mit einem Gehäuse und einer in dem Gehäuse geführten Nadel im Querschnitt; und
- Fig. 2A bis 2C: Membrane zur Verwendung in der Vorrichtung nach Fig. 1 im Querschnitt.

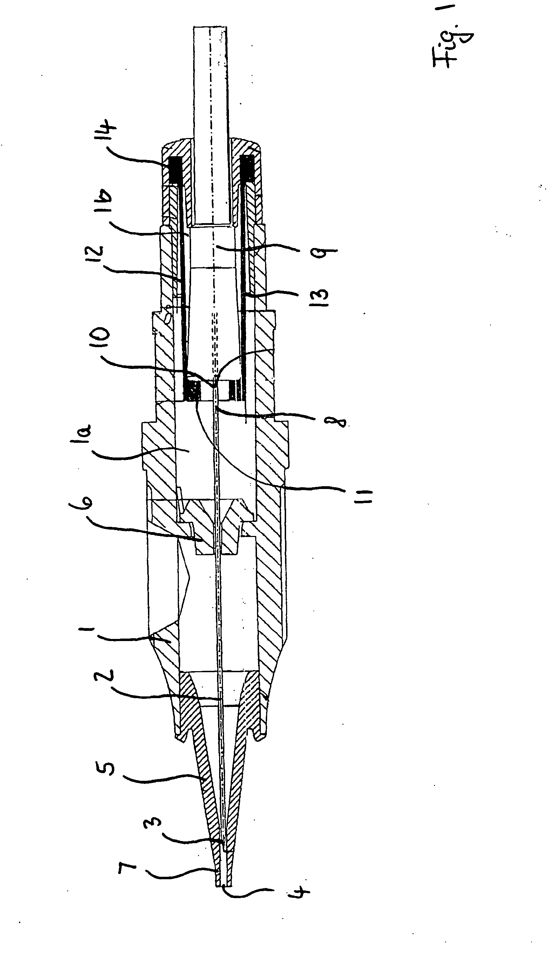

Fig. 1 zeigt eine Vorrichtung zum lokalen Aufstechen einer Haut, insbesondere zum Aufbringen von permanentem Make-up oder eines Tattoos oder zum Applizieren eines Wirkstoffs mit einem Gehäuse 1, in dem eine Nadel 2 derart geführt ist, daß die Nadel 2 zwischen einer eingefahrenen Stellung, welche in Fig. 1 dargestellt ist, und einer ausgefahrenen Stellung verlagerbar ist, in welcher eine Nadelspitze 3 außerhalb einer Öffnung 4 an einer Spitze 5 des Gehäuses 1 angeordnet ist. Es können jedoch auch Ausführungsformen vorgesehen sein, bei denen die Nadelspitze 3 der Nadel 2 auch in der eingefahrenen Stellung noch außerhalb des Gehäuses 1 aber in einer zurückgefahrenen Stellung angeordnet ist.

Wenn bei der dargestellten Ausführungsform die Nadel 2 beim Bewegen in die ausgefahrene Stellung mit der Nadelspitze 3 aus der Öffnung 4 austritt, wird die Haut lokal aufgestochen, so daß ein Medium in die Haut eindringen kann. Anschließend wird die Nadel 2 wieder zurück in das Gehäuse 1 bewegt und erneut ausgefahren. Diese Vorwärts- und Rückwärtsbewegung der Nadel 2 wird mit hoher Frequenz wiederholt. Bei der Bewegung der Nadel 2 wird diese in einer Buchse 6 geführt, welche zusammen mit einem vorderen Ende 7 der Gehäusespitze 5 als Nadelführung wirkt.

An einem rückseitigen Ende 8 der Nadel 2 ist ein Nadelschaft 9 gebildet. Die Nadel 2 ist in einem Durchbruch 10 eines mittleren Abschnitts 11 einer Membran 12 angeordnet. Die Membran 12 ist aus einem elastisch dehnbaren Material, beispielsweise Gummi oder Kunststoff. Der Durchbruch 10 ist im Querschnitt etwas kleiner als der Durchmesser die Nadel 2, so daß die Nadel 2 beim Einführen in den Durchbruch 10 geklemmt wird, was zur Abdichtung beiträgt.

Mit Hilfe der Membran 12 werden ein vorderer Inneraumabschnitt 1a auf einer Vorderseite der Membran 12 und ein hinterer Innenraumabschnitt 1b in dem Gehäuse 1 voneinander getrennt. Die Trennung mittels der Membran 12 ist vorzugsweise fluiddicht, so daß das in die Haut einzubringende flüssige Medium und/oder Körperflüssigkeit, die beim Aufstechen der Haut austritt, nicht aus dem Innenraumabschnitt 1a in den hinteren Innenraumabschnitt 1b gelangen können. Bei dem dargestellten Ausführungsbeispiel ist der hintere Innenraumabschnitt 1b in dem Gehäuse 1 gebildet. Es kann auch vorgesehen sein, daß die Membran 12 einen Abschluß des Gehäuses 1 bildet, so daß der Raumabschnitt auf der Rückseite der Membran 12, welcher bei dem Ausführungsbeispiel von dem hintere Innenraumabschnitt 1b gebildet wird, außerhalb des Gehäuses 1, in einer anzukoppelnden Antriebseinrichtung (nicht dargestellt) gebildet ist.

Beim Betrieb der Vorrichtung zum Aufstechen der Haut wird über den Nadelschaft 9 auf die Nadel 2 eine von einer Antriebseinrichtung, die auf das Gehäuse 1 beispielsweise aufgeschraubt oder aufgesteckt wird, erzeugte Antriebskraft zum Bewegen der Nadel 2 in die ausgefahrene Stellung eingeleitet. Hierbei drückt der Nadelschaft 9 auf der Rückseite gegen den mittleren Abschnitt 11 der Membran 12. Dieses führt dazu, daß die Membran 12 gedehnt wird, insbesondere im Bereich eines seitlichen Abschnitts 13, welcher im wesentlichen in Längsrichtung der Nadel 2 verläuft. Aufgrund der Dehnung der Membran 12 in Richtung des vorderen Innenraumabschnitts 1a wird von der Membran 12 eine Rückholkraft erzeugt, die über den mittleren Abschnitt 11 auf den Nadelschaft 9 und hierüber auf die Nadel 2 eingeleitet wird, so daß die Nadel 2 in Richtung der in Fig. 1 dargestellten eingefahrenen Stellung zurück bewegt wird, wenn die Antriebskraft zum Vortrieb der Nadel 2 nicht länger auf den Nadelschaft 9 wirkt. Bei der hierdurch verursachten Rückbewegung der Nadel 2 drückt der mittlere Abschnitt 11 gegen den Nadelschaft 9, so daß zwischen beiden eine feste Kopplung geschaffen ist, die dafür sorgt, daß der mittlere Abschnitt 11 und der Nadelschaft 9 aneinander gekoppelt sind und die Rückbewegung gemeinsam und in ihrer relativen Position zueinander fixiert ausführen. Die Nadel 2 wird also nicht aufgrund der Rückwärtsbewegung eines Kopplungsbauteils der Antriebseinrichtung in die eingefahrene Stellung zurückgeführt, sondern mittels der von der Membran 12 erzeugten Rückholkraft. Hierin besteht ein wesentlicher Unterschied zu bekannten Vorrichtungen, bei denen die Nadel oder der Nadelschaft von dem Kopplungsbauteil der Antriebseinrichtung zurückgezogen werden.

Um alternativ oder ergänzend zu der beschriebenen Ausführungsform eine feste Kopplung zwischen der Nadel 2 und der Membran 12 zu bilden, kann vorgesehen sein, daß auf dem Umfang der Nadel 2 eine Vertiefung gebildet ist, in welche ein Vorsprung an der Membran 12 eingreift, so daß wiederum sowohl eine dichte Trennung als auch eine feste Kopplung erreicht sind.

Um die Dichtwirkung für die Nadel 2 in dem Durchbruch 10 im Bereich des mittleren Abschnitts 11 der Membran 12 zu optimieren, ist die Membran 12 im Bereich des mittleren Abschnitts 11 dicker als im seitlichen Abschnitt 13 ausgeführt. Die Membran 12 weist am äußeren Umfang einen Halteabschnitt 14 auf, welcher an dem Gehäuse 1 zum Halten der Membran 12 befestigt ist, beispielsweise mittels Klemmwirkung. Die Membran 12 kann von dem Gehäuse 1 gelöst werden, indem ein endseitiger Gehäuseabschnitt 15 von dem Gehäuse 1 gelöst wird.

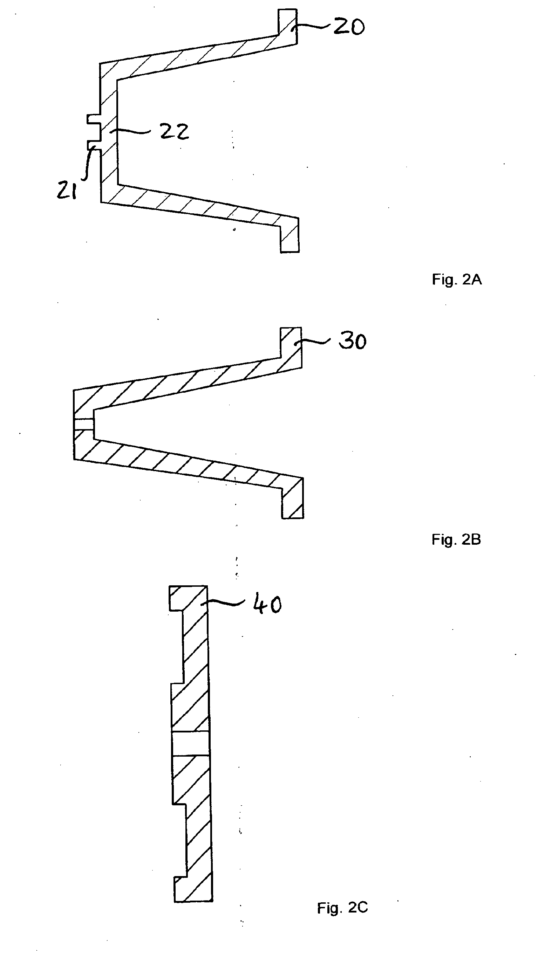

Die Figuren 2A bis 2C zeigen weitere Ausführungsformen für Membrane 20, 30, 40, die als Membran 12 bei der Vorrichtung nach Fig. 1 verwendet werden können. So kann insbesondere vorgesehen sein, daß die Membran im Querschnitt U-förmig oder V-förmig ist. In Fig. 2A ist eine Ausführungsform dargestellt, bei der im Unterschied zu der Membran 12 in Fig. 1 anstelle des Durchbruchs 10 zur Aufnahme der Nadel 2 ein vorderseitiger Montageabschnitt 21 gebildet ist, in welchen die Nadel 2 oder der Nadelschaft 9 eingesteckt werden können, um die Nadel 2 fest an die Membran 20 zu koppeln, so daß diese bei Zurückziehen der Membran 12 nach deren Streckung auch rückwärts bewegt wird in die eingefahrene Stellung. Bei dieser Ausführungsform erfolgt die Einleitung der Antriebskraft auf die Nadel 2 nicht über einem direkten Kontakt zwischen Nadel 2/ Nadelschaft 9 und Antriebseinrichtung, sondern unter Zwischenschaltung eines Membranabschnitts 22, welcher als eine Art Kupplungsteil dämpfend wirkt.

Die in der vorstehenden Beschreibung, den Ansprüchen und der Zeichnung offenbarten Merkmale der Erfindung können sowohl einzeln als auch in beliebiger Kombination für die Die in der vorstehenden Beschreibung, den Ansprüchen und der Zeichnung offenbarten Merkmale der Erfindung können sowohl einzeln als auch in beliebiger Kombination für die Verwirklichung der Erfindung in ihren verschiedenen Ausführungsformen von Bedeutung sein. Der Schutzgegenstand wird durch die zugehörigen Ansprüche definiert.

## Patentansprüche (Patentansprüche für folgende(n) Vertragsstaat(en): AT, BE, BG, CH, CY, CZ, DE, DK, EE, ES, FI, FR, GB, GR, HU, IE, IT, LI, LU, MC, NL, PL, PT, RO, SE, SI, SK, TR)

1. Vorrichtung zum lokalen Aufstechen einer Haut, insbesondere zum Aufbringen von permanentem Make-up oder eines Tattoos, mit:
- einem Gehäuse (1),
- einer in dem Gehäuse (1) geführten Nadel (2), die zwischen einer ausgefahrenen Stellung und einer eingefahrenen Stellung verlagerbar ist, und
- einer Membran (12) aus einem elastisch dehnbaren Material, wobei mit Hilfe der Membran (12) in dem Gehäuse (1) ein vorderseitiger Raum (1a) auf einer Vorderseite der Membran (12) und ein rückseitiger Raum (1b) auf einer Rückseite der Membran (12) getrennt sind,
**dadurch gekennzeichnet, daß** mit Hilfe eines Kopplungsmechanismus die Nadel (2) und die Membran (12) für eine Rückbewegung der Nadel (2) in Richtung der eingefahrenen Stellung fest miteinander gekoppelt sind, wobei über den Kopplungsmechanismus eine von der Membran (12) erzeugte Rückholkraft zum Zurückbewegen der Nadel (2) in Richtung der eingefahrenen Stellung auf die Nadel (2) eingeleitet wird.

2. Vorrichtung nach Anspruch 1, **dadurch gekennzeichnet, daß** an der Nadel (2) ein Nadelschaft (9) gebildet ist und die von der Membran (12) erzeugte Rückholkraft über den Nadelschaft (9) auf die Nadel (2) eingeleitet wird.

3. Vorrichtung nach Anspruch 1 oder 2, **dadurch gekennzeichnet, daß** der Kopplungsmechanismus an der Membran gebildete Montagemittel (21) zum Halten der Nadel (2) auf der Vorderseite der Membran (12) umfaßt, in welchen ein Ende der Nadel (2) montiert ist.

4. Vorrichtung nach einem der vorangehenden Ansprüche, **dadurch gekennzeichnet, daß** der vorderseitige Raum (1a) auf der Vorderseite der Membran (12) und der rückseitige Raum (1b) auf der Rückseite der Membran (12) mit Hilfe der Membran (12) fluiddicht getrennt sind.

5. Vorrichtung nach einem der vorangehenden Ansprüche, **dadurch gekennzeichnet, daß** die Membran (12) dehnbare Abschnitte (14) aufweist, die in einer nicht gedehnten Grundform der Membran (12) im wesentlichen in Längsrichtung der Nadel (2) gebildet sind.

6. Vorrichtung nach Anspruch 5, **dadurch gekennzeichnet, daß** ein Querschnitt der Membran (12) in Längsrichtung U-förmig oder V-förmig ist.

7. Vorrichtung nach einem der vorangehenden Ansprüche, **dadurch gekennzeichnet, daß** die Membran (12) lösbar an dem Gehäuse (1) montiert ist.

8. Vorrichtung nach einem der vorangehenden Ansprüche, **dadurch gekennzeichnet, daß** das Gehäuse (1) als ein Modul ausgeführt ist, welches lösbar an ein Antriebsmodul koppelbar ist.

9. Vorrichtung nach Anspruch 8, **dadurch gekennzeichnet, daß** das Modul ein Einwegmodul ist.

10. Verwendung einer Vorrichtung nach einem der vorangehenden Ansprüche zum Aufbringen von permanentem Make-up oder eines Tattoos auf eine Haut.

## Patentansprüche (Patentansprüche für folgende(n) Vertragsstaat(en): DE)

1. Vorrichtung zum lokalen Aufstechen einer Haut, insbesondere zum Aufbringen von permanentem Make-up oder eines Tattoos, mit:
- einem Gehäuse (1),
- einer in dem Gehäuse (1) geführten Nadel (2), die zwischen einer ausgefahrenen Stellung und einer eingefahrenen Stellung verlagerbar ist, und
- einer Membran (12) aus einem elastisch dehnbaren Material, wobei mit Hilfe der Membran (12) in dem Gehäuse (1) ein vorderseitiger Raum (1a) auf einer Vorderseite der Membran (12) und ein rückseitiger Raum (1b) auf einer Rückseite der Membran (12) getrennt sind,
wobei mit Hilfe eines Kopplungsmechanismus die Nadel (2) und die Membran (12) für eine Rückbewegung der Nadel (2) in Richtung der eingefahrenen Stellung fest miteinander gekoppelt sind und wobei über den Kopplungsmechanismus eine von der Membran (12) erzeugte Rückholkraft zum Zurückbewegen der Nadel (2) in Richtung der eingefahrenen Stellung auf die Nadel (2) eingeleitet wird, **dadurch gekennzeichnet, daß** die Membran (12) dehnbare Abschnitte (14) aufweist, die in einer nicht gedehnten Grundform der Membran (12) im wesentlichen in Längsrichtung der Nadel (2) gebildet sind.

2. Vorrichtung nach Anspruch 1, **dadurch gekennzeichnet, daß** an der Nadel (2) ein Nadelschaft (9) gebildet ist und die von der Membran (12) erzeugte Rückholkraft über den Nadelschaft (9) auf die Nadel (2) eingeleitet wird.

3. Vorrichtung nach Anspruch 1 oder 2, **dadurch gekennzeichnet, daß** der Kopplungsmechanismus an der Membran gebildete Montagemittel (21) zum Halten der Nadel (2) auf der Vorderseite der Membran (12) umfaßt, in welchen ein Ende der Nadel (2) montiert ist.

4. Vorrichtung nach einem der vorangehenden Ansprüche, **dadurch gekennzeichnet, daß** der vorderseitige Raum (1a) auf der Vorderseite der Membran (12) und der rückseitige Raum (1b) auf der Rückseite der Membran (12) mit Hilfe der Membran (12) fluiddicht getrennt sind.

5. Vorrichtung nach Anspruch 1, **dadurch gekennzeichnet, daß** ein Querschnitt der Membran (12) in Längsrichtung U-förmig oder V-förmig ist.

6. Vorrichtung nach einem der vorangehenden Ansprüche, **dadurch gekennzeichnet, daß** die Membran (12) lösbar an dem Gehäuse (1) montiert ist.

7. Vorrichtung nach einem der vorangehenden Ansprüche, **dadurch gekennzeichnet, daß** das Gehäuse (1) als ein Modul ausgeführt ist, welches lösbar an ein Antriebsmodul koppelbar ist.

8. Vorrichtung nach Anspruch 7, **dadurch gekennzeichnet, daß** das Modul ein Einwegmodul ist.

9. Verwendung einer Vorrichtung nach einem der vorangehenden Ansprüche zum Aufbringen von permanentem Make-up oder eines Tattoos auf eine Haut.

## Claims (Claims for the following Contracting State(s): AT, BE, BG, CH, CY, CZ, DE, DK, EE, ES, FI, FR, GB, GR, HU, IE, IT, LI, LU, MC, NL, PL, PT, RO, SE, SI, SK, TR)

1. A device for local puncturing of a skin, in particular for applying permanent make-up or a tattoo, comprising:
- a housing (1),
- a needle (2) guided in the housing (1), movable between an extended position and a retracted position, and
- a diaphragm (12) made of an elastically extensible material and dividing a front-side space (1a) at a front side of the diaphragm (12) from a rear-side space (1b) at a back side of the diaphragm (12) in the housing (1),
**characterized in that** a coupling mechanism firmly couples the needle (2) and the diaphragm (12) for a backward movement of the needle (2) in the direction of the retracted position, a return force generated by the diaphragm (12) for moving the needle (2) back in the direction of the retracted position being introduced onto the needle by the coupling mechanism.

2. The device as claimed in claim 1, **characterized in that** the needle (2) is formed with a needle shaft (9) and the return force generated by the diaphragm (12) is introduced onto the needle (2) through the needle shaft (9).

3. The device as claimed in claim 1 or 2, **characterized in that** the coupling mechanism includes mounting means (21) formed on the diaphragm at the front surface of the diaphragm (12) to retain the needle (2), one end of the needle (2) being mounted in said mounting means.

4. The device as claimed in any one of the preceding claims, **characterized in that** the front-side space (1a) at the front side of the diaphragm (12) and the rear-side space (1b) at the back side of the diaphragm (12) are separated in fluid tight fashion by the diaphragm (12).

5. The device as claimed in any one of the preceding claims, **characterized in that** the diaphragm (12) comprises extensible portions (14) formed essentially in longitudinal direction of the needle (2), in a non-extended basic configuration of the diaphragm (12).

6. The device as claimed in claim 5, **characterized in that** a cross section of the diaphragm (12) is U-shaped or V-shaped in longitudinal direction.

7. The device as claimed in any of the preceding claims, **characterized in that** the diaphragm (12) is mounted releasably at the housing (1).

8. The device as claimed in any one of the preceding claims, **characterized in that** the housing (1) is devised as a module for releasable coupling to a drive module.

9. The device as claimed in claim 8, **characterized in that** the module is a one-way module.

10. Use of the device as claimed in any one of the preceding claims for applying permanent make-up or a tattoo to skin.

## Claims (Claims for the following Contracting State(s): DE)

1. A device for local puncturing of a skin, in particular for applying permanent make-up or a tattoo, comprising:
- a housing (1),
- a needle (2) guided in the housing (1), movable between an extended position and a retracted position, and
- a diaphragm (12) made of an elastically extensible material and dividing a front-side space (1a) at a front side of the diaphragm (12) from a rear-side space (1b) at a back side of the diaphragm (12) in the housing (1),
wherein a coupling mechanism firmly couples the needle (2) and the diaphragm (12) for a backward movement of the needle (2) in the direction of the retracted position, a return force generated by the diaphragm (12) for moving the needle (2) back in the direction of the retracted position being introduced onto the needle by the coupling mechanism, **characterized in that** the diaphragm (12) comprises extensible portions (14) formed essentially in longitudinal direction of the needle (2), in a non-extended basic configuration of the diaphragm (12).

2. The device as claimed in claim 1, **characterized in that** the needle (2) is formed with a needle shaft (9) and the return force generated by the diaphragm (12) is introduced onto the needle (2) through the needle shaft (9).

3. The device as claimed in claim 1 or 2, **characterized in that** the coupling mechanism includes mounting means (21) formed on the diaphragm at the front surface of the diaphragm (12) to retain the needle (2), one end of the needle (2) being mounted in said mounting means.

4. The device as claimed in any one of the preceding claims, **characterized in that** the front-side space (1a) at the front side of the diaphragm (12) and the rear-side space (1b) at the back side of the diaphragm (12) are separated in fluid tight fashion by the diaphragm (12).

5. The device as claimed in any of the preceding claims, **characterized in that** a cross section of the diaphragm (12) is U-shaped or V-shaped in longitudinal direction.

6. The device as claimed in any of the preceding claims, **characterized in that** the diaphragm (12) is mounted releasably at the housing (1).

7. The device as claimed in any one of the preceding claims, **characterized in that** the housing (1) is devised as a module for releasable coupling to a drive module.

8. The device as claimed in claim 7, **characterized in that** the module is a one-way module.

9. Use of the device as claimed in any one of the preceding claims for applying permanent make-up or a tattoo to skin.

## Revendications (Revendications pour l'(les) Etat(s) contractant(s) suivant(s): AT, BE, BG, CH, CY, CZ, DE, DK, EE, ES, FI, FR, GB, GR, HU, IE, IT, LI, LU, MC, NL, PL, PT, RO, SE, SI, SK, TR)

1. Dispositif servant à piquer localement la peau, en particulier pour l'application d'un maquillage permanent ou d'un tatouage, comprenant :
- un boîtier (1),
- une aiguille (2) guidée dans le boîtier (1), aiguille qui peut être déplacée entre une position sortie et une position rentrée, et
- une membrane (12) fabriquée dans une matière élastiquement extensible, où un espace avant (1a) placé sur une face avant de la membrane (12) et un espace arrière (1b) placé sur une face arrière de la membrane (12) sont séparés, dans le boîtier (1), à l'aide de la membrane (12),
**caractérisé en ce que** l'aiguille (2) et la membrane (12) sont fixement couplées l'une à l'autre, à l'aide d'un mécanisme de couplage, pour un mouvement arrière de l'aiguille (2) en direction de la position rentrée, où une force de rappel produite par la membrane (12), pour le retour de l'aiguille (2) en direction de la position rentrée, est appliquée sur l'aiguille (2) via le mécanisme de couplage.

2. Dispositif selon la revendication 1,
**caractérisé en ce qu'**une tige d'aiguille (9) est formée sur l'aiguille (2) et la force de rappel produite par la membrane (12) est appliquée sur l'aiguille (2) via la tige (9) de l'aiguille.

3. Dispositif selon la revendication 1 ou 2, **caractérisé en ce que** le mécanisme de couplage comprend des moyens de montage (21) formés sur la membrane et servant à retenir l'aiguille (2) sur la face avant de la membrane (12), moyens de montage dans lesquels est montée une extrémité de l'aiguille (2).

4. Dispositif selon l'une quelconque des revendications précédentes, **caractérisé en ce que** l'espace avant (1a) placé sur la face avant de la membrane (12) et l'espace arrière (1b) placé sur la face arrière de la membrane (12) sont séparés en étant étanches au fluide, à l'aide de la membrane (12).

5. Dispositif selon l'une quelconque des revendications précédentes, **caractérisé en ce que** la membrane (12) présente des parties extensibles (14) qui sont formées dans une forme de base, non allongée, de la membrane (12), pratiquement suivant la direction longitudinale de l'aiguille (2).

6. Dispositif selon la revendication 5, **caractérisé en ce qu'**une coupe transversale de la membrane (12) est, dans la direction longitudinale, en forme de U ou en forme de V.

7. Dispositif selon l'une quelconque des revendications précédentes, **caractérisé en ce que** la membrane (12) est montée de façon amovible sur le boîtier (1).

8. Dispositif selon l'une quelconque des revendications précédentes, **caractérisé en ce que** le boîtier (1) est réalisé comme un module qui peut, de façon détachable, être couplé à un module d'entraînement.

9. Dispositif selon la revendication 8, **caractérisé en ce que** le module est un module à usage unique.

10. Utilisation d'un dispositif selon l'une quelconque des revendications précédentes, servant à l'application, sur la peau, d'un maquillage permanent ou d'un tatouage.

## Revendications (Revendications pour l'(les) Etat(s) contractant(s) suivant(s): DE)

1. Dispositif servant à piquer localement la peau, en particulier pour l'application d'un maquillage permanent ou d'un tatouage, comprenant
- un boîtier (1),
- une aiguille (2) guidée dans le boîtier (1), aiguille qui peut être déplacée entre une position sortie et une position rentrée, et
- une membrane (12) fabriquée dans une matière élastiquement extensible, où un espace avant (1a) placé sur une face avant de la membrane (12) et un espace arrière (1b) placé sur une face arrière de la membrane (12) sont séparés, dans le boîtier (1), à l'aide de la membrane (12),
et où l'aiguille (2) et la membrane (12) sont fixement couplées l'une à l'autre, à l'aide d'un mécanisme de couplage, pour un mouvement arrière de l'aiguille (2) en direction de la position rentrée, où une force de rappel produite par la membrane (12), pour le retour de l'aiguille (2) en direction de la position rentrée, est appliquée sur l'aiguille (2) via le mécanisme de couplage, **caractérisé en ce que** la membrane (12) présente des parties extensibles (14) qui sont formées dans une forme de base, non allongée, de la membrane (12) pratiquement suivant la direction longitudinale de l'aiguille (2).

2. Dispositif selon la revendication 1, **caractérisé en ce qu'**une tige d'aiguille (9) est formée sur l'aiguille (2) et la force de rappel produite par la membrane (12) est appliquée sur l'aiguille (2) via la tige (9) de l'aiguille.

3. Dispositif selon la revendication 1 ou 2, **caractérisé en ce que** le mécanisme de couplage comprend des moyens de montage (21) formés sur la membrane et servant à retenir l'aiguille (2) sur la face avant de la membrane (12), moyens de montage dans lesquels est montée une extrémité de l'aiguille (2).

4. Dispositif selon l'une quelconque des revendications précédentes, **caractérisé en ce que** l'espace avant (1a) placé sur la face avant de la membrane (12) et l'espace arrière (1b) placé sur la face arrière de la membrane (12) sont séparés en étant étanches au fluide, à l'aide de la membrane (12).

5. Dispositif selon l'une quelconque des revendications précédentes, **caractérisé en ce qu'**une coupe transversale de la membrane (12) est, dans la direction longitudinale, en forme de U ou en forme de V.

6. Dispositif selon l'une quelconque des revendications précédentes, **caractérisé en ce que** la membrane (12) est montée de façon amovible sur le boîtier (1).

7. Dispositif selon l'une quelconque des revendications précédentes, **caractérisé en ce que** le boîtier (1) est réalisé comme un module qui peut, de façon détachable, être couplé un module d'entraînement.

8. Dispositif selon la revendication 7, **caractérisé en ce que** le module est un module à usage unique.

9. Utilisation d'un dispositif selon l'une quelconque des revendications précédentes, servant à l'application, sur la peau, d'un maquillage permanent ou d'un tatouage.
